# EUROPEAN PATENT SPECIFICATION

(11) **EP 3 804 677 B1**
(45) Date of publication and mention of the grant of the patent: **11.10.2023**
(21) Application number: 19827179.3
(22) Date of filing: 14.06.2019
(51) Int. Cl.: A61F 13/496, A61F 13/49

(54) **ABSORBENT ARTICLE**
ABSORBIERENDER ARTIKEL
ARTICLE ABSORBANT

(30) Priority: 29.06.2018 JP 2018124677
(43) Date of publication of application: 14.04.2021
(73) Proprietor: Unicharm Corporation, Shikokuchuo-shi, Ehime 799-0111 (JP)
(72) Inventor: OKUNO, Shingo, Kanonji-shi, Kagawa 769-1602 (JP); ICHIKAWA, Makoto, Kanonji-shi, Kagawa 769-1602 (JP); KAWABATA, Kuniyoshi, Kanonji-shi, Kagawa 769-1602 (JP)
(74) Representative: Dolleymores
(86) International application number: PCT/JP2019/023724
(87) International publication number: WO 2020/004081

(56) References cited:
- JP-A- H11 332 912
- JP-A- 2009 509 620
- JP-A- 2013 146 419
- JP-A- 2013 233 383
- JP-A- 2015 146 887

## Description

### [Technical Field]

The present disclosure relates to an absorbent article.

### [Background Art]

There is known a pants-type disposable diaper including a stomach-side portion and a back-side portion, which are joined to each other by a joining portion, and the joining portion is formed by joining an end portion of the stomach-side portion in the lateral direction and an end portion of the back-side portion in the lateral direction, which are overlapped on each other such that inner surfaces face each other with a plurality of fusion portions aligned in the longitudinal direction at intervals (for example, refer to Patent Literature 1 to 5). Patent Literature 6 discloses a shorts-type diaper where both side edge parts of the front body part and the rear body part are joined.

### [Patent Literature]

Patent Literature 1: Japanese Unexamined Patent Publication No. 2016-107117
Patent Literature 2: Japanese Unexamined Patent Publication No. 2013-146419
Patent Literature 3: JP 2015-146887 A
Patent Literature 4: JP 2013 - 233383 A
Patent Literature 5: JP 2009-509620 A
Patent Literature 6: JP H11-332912 A

### [Summary of the Invention]

### [Technical Problem]

When a wearer wears a pants-type diaper for himself or herself or a caregiver puts a pants-type diaper on a dependent, it is assumed that the wearer or the caregiver grasps, in particular, a portion around the waist opening of the pants-type diaper and pulls up the pants-type diaper. Specifically, it is considered that the wearer, for example, positions the thumb on the inner side of the pants-type diaper and positions the index finger and the like on the outer side of the pants-type diaper, thereby pinching the pants-type diaper with these fingers. However, at this time, there is a concern that the index finger or the like of the wearer or the caregiver may touch the joining portion. There are a plurality of fusion portions in the joining portion, and these fusion portions are relatively hard. Therefore, there is a concern that the wearer or the caregiver may feel discomfort when wearing the pants-type diaper or putting the pants-type diaper on. Alternatively, before the wearer wears clothes on the pants-type diaper or the caregiver puts clothes on the pants-type diaper after the pants-type diaper has been put on, the same problem may also occur when the arm or the like of the wearer or the caregiver touches the joining portion. Regarding this point, it might be possible to restrict the discomfort that the wearer or the caregiver feels by, for example, reducing the area of the fusion portions. However, when the area of the fusion portions is reduced, there is a concern that the joining strength of the joining portion may decrease.

### [Solution to Problem]

The present invention provides the absorbent article of independent claim 1. The dependent claims specify preferred but optional features.

According to the present disclosure, the following configurations are provided.

### [Configuration 1]

An absorbent article including a stomach-side portion and a back-side portion that are joined to each other with at least one joining portion, in which the joining portion is formed by joining an end portion of the stomach-side portion in a lateral direction and an end portion of the back-side portion in the lateral direction that are overlapped on each other such that inner surfaces face each other with a plurality of fusion portions aligned in a longitudinal direction at intervals, each of the joining portion is divided into an upper joining part positioned on an upper side and a lower joining part positioned on a lower side in the longitudinal direction, the joining portion is elastic in the lateral direction, tensile stress in the lateral direction in the upper joining part is larger than tensile stress in the lateral direction in the lower joining part, the joining portion includes the fusion portions and a margin present on an outer side of the fusion portions in the lateral direction, a dimension of the margin in the lateral direction in the upper joining part is larger than a dimension of the margin in the lateral direction in the lower joining part, and an area of each of the fusion portions positioned in the upper joining part is larger than an area of each of the fusion portions positioned in the lower joining part. According to Configuration 1, in the upper joining part that a wearer or a caregiver is highly likely to touch, the dimension of the margin in the lateral direction is relatively large. As will be described below in detail, the margin is capable of covering the fusion portions. Therefore, the margin restricts the wearer or the caregiver from directly touching the joining portion or the fusion portions. In addition, according to Configuration 1, the tensile stress in the lateral direction in the upper joining part is relatively large. As a result, the margin is maintained near the fusion portions in a contracted state. That is, a state in which the fusion portions are covered with the margin is maintained. Furthermore, the margin further contracts by the tensile stress in the lateral direction, whereby the cushioning property of the margin is enhanced. Therefore, the wearer or the caregiver is restricted from feeling discomfort when touching the joining portion. Moreover, the area of each of the fusion portions positioned in the upper joining part is relatively large. Therefore, the wearer or the caregiver is restricted from feeling discomfort when touching the joining portion while maintaining the joining strength in the upper joining part relatively high.

### [Configuration 2]

The absorbent article according to Configuration 1, in which, in the upper joining part, the dimension of the margin in the lateral direction is equal to or larger than a dimension of the fusion portion in the lateral direction. According to Configuration 2, the margin is made larger in the upper joining part. As a result, in the upper joining part, the fusion portions are more reliably covered with the margin. Therefore, the wearer or the caregiver is further restricted from feeling discomfort when touching the joining portion while maintaining the joining strength in the upper joining part relatively high.

### [Configuration 3]

The absorbent article according to Configuration 1 or 2, in which an occupancy of the fusion portions in the upper joining part is smaller than an occupancy of the fusion portions in the lower joining part. According to Configuration 3, in the upper joining part, the occupancy of the relatively hard fusion portions is relatively small. As a result, the feeling at the time of touching the upper joining part becomes more flexible. Therefore, the wearer or the caregiver is further restricted from feeling discomfort when touching the joining portion while maintaining the joining strength in the upper joining part relatively high.

### [Configuration 4]

The absorbent article according to any one of Configurations 1 to 3, in which a dimension of the fusion portion in the lateral direction in the upper joining part is smaller than a dimension of the fusion portion in the lateral direction in the lower joining part. According to Configuration 4, it becomes possible to make the margin larger in the upper joining part. As a result, in the upper joining part, the fusion portions are more reliably covered with the margin. Therefore, the wearer or the caregiver is further restricted from feeling discomfort when touching the joining portion while maintaining the joining strength in the upper joining part relatively high.

### [Configuration 5]

The absorbent article according to any one of Configurations 1 to 4, in which an outline of the fusion portion in the upper joining part has a curved shape throughout an entire circumference. According to Configuration 5, it becomes possible for the contracted margin to more easily come close to the fusion portions. Therefore, the wearer or the caregiver is further restricted from feeling discomfort when touching the joining portion while maintaining the joining strength in the upper joining part relatively high.

### [Configuration 6]

The absorbent article according to any one of Configurations 1 to 5, in which, in the upper joining part, a dimension of the fusion portion in the longitudinal direction is equal to or smaller than a dimension of the fusion portion in the lateral direction. According to Configuration 6, the flexibility in the longitudinal direction of the upper joining part is further enhanced. Therefore, the wearer or the caregiver is further restricted from feeling discomfort when touching the joining portion while maintaining the joining strength in the upper joining part relatively high.

### [Configuration 7]

The absorbent article according to any one of Configurations 1 to 6, in which the absorbent article is for adults. According to Configuration 7, in the absorbent article for adults, the wearer or the caregiver is further restricted from feeling discomfort when touching the joining portion while maintaining the joining strength in the upper joining part relatively high.

### [Advantageous Effect of the Invention]

It is possible to restrict wearers or caregivers from feeling discomfort when touching the joining portion while maintaining the joining strength in the upper joining part relatively high.

### [Brief Description of Drawings]

Fig. 1 is a perspective view of a disposable diaper of an embodiment according to the present disclosure in a worn state.
Fig. 2 is a plan view of the disposable diaper of Fig. 1 in an unfolded state.
Fig. 3 is a schematic view of end portions of a stomach-side portion and a back-side portion in a lateral direction viewed from the lateral direction.
Fig. 4 is a partial plan view of a disposable diaper in an extended state according to a reference example which does not form part of the present invention.
Fig. 5 is a partial enlarged view of a joining portion.
Fig. 6 is a schematic view of the joining portion when the disposable diaper is worn that is viewed from above in a longitudinal direction.
Fig. 7 is an enlarged plan view showing a joining portion of another embodiment according to the present disclosure.
Fig. 8(A) is an enlarged view of a part A in Fig. 7, Fig. 8(B) is an enlarged view of a part B in Fig. 7, and Fig. 8(C) is an enlarged view of a part C in Fig. 7.
Fig. 9 is a partial plan view, which is similar to Fig. 4, showing another embodiment according to the present disclosure.
Fig. 10 is a partial plan view, which is similar to Fig. 4, showing another reference example which does not form part of the present invention.

### [Description of Embodiments]

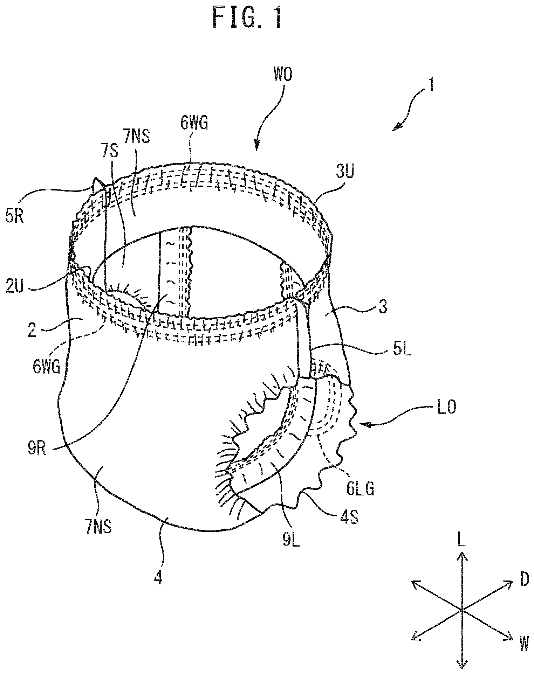

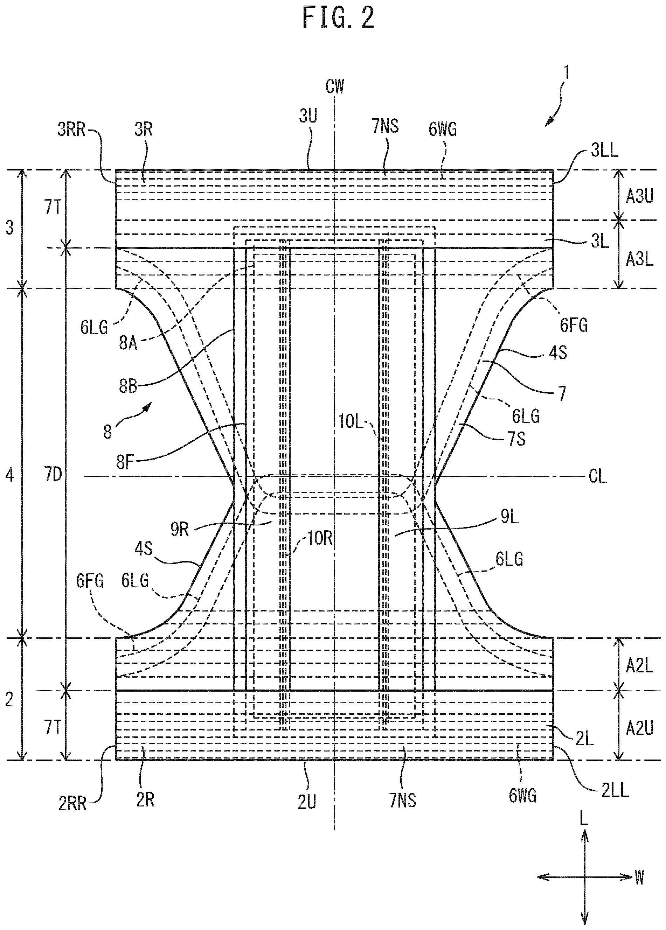

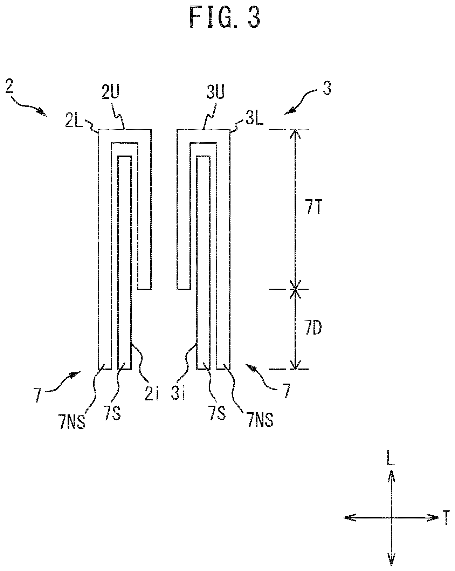

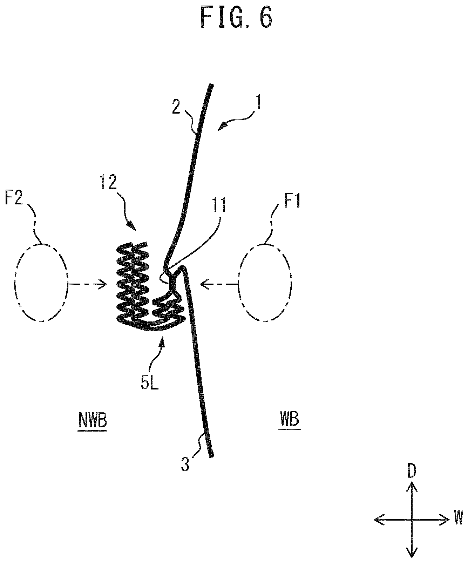

Fig. 1 and Fig. 2 show an absorbent article 1 of an embodiment according to the present disclosure. In the embodiment according to the present disclosure, the absorbent article 1 is comprised of a pants-type disposable diaper (hereinafter, also simply referred to as "disposable diaper"). In Fig. 2, L represents the longitudinal direction of the disposable diaper 1 in an unfolded state, and W represents the lateral direction of the disposable diaper 1 that is perpendicular to the longitudinal direction L, respectively. In addition, in Fig. 2, CL represents a longitudinal direction centerline that passes through the center of the disposable diaper 1 in the longitudinal direction L and extends in the lateral direction W, and CW represents a lateral direction centerline that passes through the center of the disposable diaper 1 in the lateral direction W and extends in the longitudinal direction L, respectively. In this case, in the longitudinal direction L, a direction and a side toward the longitudinal direction centerline CL are the inward direction and the inner side, and a direction and a side away from the longitudinal direction centerline CL are the outward direction and the outer side. In the lateral direction W, a direction and a side toward the lateral direction centerline CW are the inward direction and the inner side, and a direction and a side away from the lateral direction centerline CW are the outward direction and the outer side. L in Fig. 1 shows the longitudinal direction of the disposable diaper 1 in a worn state. The downward direction or the lower side in the longitudinal direction L in Fig. 1 corresponds to the inward direction and the inner side in the longitudinal direction L in Fig.2, and the upward direction or the upper side in the longitudinal direction L in Fig. 1 corresponds to the outward direction and the outer side in the longitudinal direction L in Fig.2. The above description shall apply to Fig. 3 to Fig. 10. In addition, D in Figs. 1 and 6 shows the depth direction of the disposable diaper 1.

Referring to Fig. 1, the disposable diaper 1 of the embodiment according to the present disclosure includes a stomach-side portion 2, a back-side portion 3, and an intermediate portion 4 between the stomach-side portion 2 and the back-side portion 3. In addition, the stomach-side portion 2 and the back-side portion 3 are joined to each other with a pair of joining portions 5R and 5L positioned at both ends of the stomach-side portion 2 and the back-side portion 3 in the lateral direction W. In this case, an upper edge 2U of the stomach-side portion 2 and an upper edge 3U of the back-side portion 3 define a single waist opening WO through which a wearer's upper limb passes. In addition, both side edges 4S and 4S of the intermediate portion 4 in the lateral direction W define a pair of leg openings LO and LO through which the wearer's legs pass. While the disposable diaper is worn, the stomach-side portion 2 is positioned to face the abdomen portion of the wearer, the back-side portion 3 is positioned to face the back portion or buttock portion of the wearer, and the intermediate portion 4 is positioned to face the crotch portion of the wearer.

In the embodiment according to the present disclosure, in an unfolded state shown in Fig. 2, the stomach-side portion 2 and the back-side portion 3 each have a basically rectangular shape that extends in the lateral direction W and are positioned apart from each other in the longitudinal direction L. In the embodiment shown in Fig. 2, the upper edge 2U of the stomach-side portion 2 and the upper edge 3U of the back-side portion 3 extend in the lateral direction W. In addition, in the embodiment shown in Fig. 2, outer edges 2LL and 2RR of the stomach-side portion 2 in the lateral direction W and outer edges 3LL and 3RR of the back-side portion 3 in the lateral direction W extend in the longitudinal direction L. The intermediate portion 4 is positioned between the stomach-side portion 2 and the back-side portion 3. Both side edges 4S and 4S of the intermediate portion 4 in the lateral direction W are recessed inward in the lateral direction W. In the embodiment according to the present disclosure, the stomach-side portion 2, the back-side portion 3, and the intermediate portion 4 are integrally formed with one another. In another embodiment (not shown), the stomach-side portion 2, the back-side portion 3, and the intermediate portion 4 are separately formed from one another.

In the embodiment according to the present disclosure, a plurality of waist gather (WG) elastic members 6WG, which are separated from each other in the longitudinal direction L with extending in the lateral direction W, are provided in an extended state in an upper region A2U adjacent to the upper edge or outer edge 2U of the stomach-side portion 2, and also provided in an upper region A3U adjacent to the upper edge or outer edge 3U of the back-side portion 3, respectively. The WG elastic members 6WG of the embodiment according to the present disclosure are provided between one outer edge 2LL of the stomach-side portion 2 in the lateral direction W and the other outer edge 2RR in the lateral direction W and between one outer edge 3LL of the back-side portion 3 in the lateral direction W and the other outer edge 3RR in the lateral direction W. In addition, a plurality of fit gather (FG) elastic members 6FG, which are separated from each other in the longitudinal direction L with extending in the lateral direction W, are provided in an extended state in a lower region A2L on the inner side of the upper region A2U of the stomach-side portion 2 in the longitudinal direction L, and also provided in a lower region A3L on the inner side of the upper region A3U of the back-side portion 3, respectively. The FG elastic members 6FG of the embodiment according to the present disclosure are provided between one outer edge 2LL of the stomach-side portion 2 in the lateral direction W and the other outer edge 2RR in the lateral direction W and between one outer edge 3LL of the back-side portion 3 in the lateral direction W and the other outer edge 3RR in the lateral direction W. Furthermore, a plurality of leg gather (LG) elastic members 6LG are provided in an extended state to be separated from each other in a direction perpendicular to an extending direction. The LG elastic members 6LG includes elastic members that extend from one outer edge 2LL of the stomach-side portion 2 in the lateral direction W, which is included in the lower region A2L of the stomach-side portion 2, along one side edge 4S of the intermediate portion 4, then, extend in the lateral direction W at the center in the lateral direction W, then, extend along the other side edge 4S of the intermediate portion 4, and reach the other outer edge 2RR of the stomach-side portion 2 in the lateral direction W, which is included in the lower region A2L of the stomach-side portion 2, and also includes elastic members that extend from one outer edge 3LL of the back-side portion 3 in the lateral direction W, which is included in the lower region A3L of the back-side portion 3, along one side edge 4S of the intermediate portion 4, then, extend in the lateral direction W at the center in the lateral direction W, then, extend along the other side edge 4S of the intermediate portion 4, and reach the other outer edge 3RR of the back-side portion 3 in the lateral direction W, that is included in the lower region A3L of the back-side portion 3.

On the upper regions A2U and A3U, tensile stress in the lateral directions W acts due mainly to the WG elastic members 6WG. On the lower regions A2L and A3L, tensile stress in the lateral directions W acts due mainly to the FG elastic members 6FG. In the embodiment according to the present disclosure, the tensile stress in the lateral direction W in the upper regions A2U and A3U is larger than the tensile stress in the lateral direction W in the lower regions A2L and A3L. In order to achieve this, for example, the extension ratios, numbers, thicknesses, cross-sectional shapes, and the like of the WG elastic members 6WG, the FG elastic members 6FG, and the LG elastic members 6LG are adjusted.

In one example, as the WG elastic members 6WG, a plurality of rubber threads of 620 dtx are provided at an extension ratio of 2.8 times and intervals of 6 mm. As the FG elastic members 6G, a plurality of rubber threads of 470 to 940 dtx are provided at an extension ratio of 2.1 to 2.7 times at intervals of 7 mm to 15mm. As the LG elastic members 6LG, a plurality of rubber threads of 620 dtx are provided at an extension ratio of 2.0 times at intervals of 7.5mm.

In the embodiment according to the present disclosure, the stomach-side portion 2, the back-side portion 3, and the intermediate portion 4 are formed of a liquid-impermeable cover sheet 7. The cover sheet 7 of the embodiment according to the present disclosure includes a skin side sheet 7S and a non-skin side sheet 7NS, which are overlapped and glued to each other. In a state where the disposable diaper is worn, the skin side sheet 7S is positioned on a side close to the wearer or on the inner side, and the non-skin side sheet 7NS is positioned on a side far from the wearer or on the outer side. In addition, in the embodiment according to the present disclosure, at both outer ends in the longitudinal direction L, the non-skin side sheet 7NS is folded back onto the skin side sheet 7S. As a result, in both outer side portions of the cover sheet 7 in the longitudinal direction L, three-layer portions 7T and 7T, in which the non-skin side sheet 7NS, the skin side sheet 7S, and the non-skin side sheet 7NS are sequentially overlapped, are formed. The three-layer portions 7T and 7T are included in the stomach-side portion 2 and the back-side portion 3. Meanwhile, in the central part of the cover sheet 7 in the longitudinal direction, a two-layer portion 7D, in which the skin side sheet 7S and the non-skin side sheet 7NS are overlapped, is formed. The above-described elastic members 6WG, 6FG, and 6LG are glued between the skin side sheet 7S and the non-skin side sheet 7NS.

The cover sheet 7 is formed of any liquid-impermeable sheet, including an impermeable non-woven fabric or synthetic resin film which is formed of, for example, a polyolefin-based material such as polypropylene or polyethylene or the like, a composite sheet thereof, an SB non-woven fabric, and an SMS non-woven fabric, or the like. In addition, the basis weight of the cover sheet 7 is, for example, 5 to 100 g/m² and preferably 10 to 50 g/m². The thickness of the cover sheet 7 is, for example, 0.2 to 5 mm and preferably 0.2 to 2 mm. In another embodiment (not shown), the cover sheet 7 is formed of a single sheet. In addition, in another embodiment (not shown), the three-layer portion is not provided, and the cover sheet 7 is entirely formed of a two-layer portion.

The disposable diaper 1 of the embodiment according to the present disclosure further includes an absorbent body 8 provided on the inner surface of the cover sheet 7. The absorbent body 8 includes a liquid-permeable top sheet 8F, a liquid-impermeable back sheet 8B, and an absorber 8A positioned between the top sheet 8F and the back sheet 8B. The absorber 8A of the embodiment according to the present disclosure includes, in the present embodiment, an absorber core and a core wrap that encloses the absorber core. The absorber 8A and the top sheet 8F and the absorber 8A and the back sheet 8B are respectively glued to each other, and the top sheet 8F and the back sheet 8B are glued to each other at their circumferential edge portions. The top sheet 8F is formed of, for example, a liquid-permeable non-woven fabric or woven fabric, a synthetic resin film in which liquid permeable holes are formed, a composite sheet thereof, or the like. The back sheet 8B is formed of, for example, a liquid-impermeable non-woven fabric or synthetic resin film, a composite sheet thereof, an SMS non-woven fabric, or the like. The absorber 8A is formed of a pulp fiber, a synthetic fiber, an absorbent polymer, or the like.

The disposable diaper 1 of the embodiment according to the present disclosure further includes liquid-impermeable, leakproof walls 9R and 9L. These leakproof walls 9R and 9L are disposed along the longitudinal direction L on both sides of the top sheet 8F in the lateral direction W. In addition, at the distal ends of the leakproof walls 9R and 9L, elastic members 10R and 10L that respectively extending in the longitudinal direction L are disposed.

In the embodiment according to the present disclosure, the disposable diaper 1 in an unfolded state is folded, for example, along the longitudinal direction centerline CL such that an inner surface 2i of the stomach-side portion 2 and an inner surface 3i of the back-side portion 3 face each other. In this case, one end 2L of the stomach-side portion 2 in the lateral direction W and one end 3L of the back-side portion 3 in the lateral direction W are overlapped on each other. Fig. 3 schematically shows one end 2L of the stomach-side portion 2 in the lateral direction W and one end 3L of the back-side portion 3 in the lateral direction W overlapped on each other. T in Fig. 3 indicates the thickness direction of the disposable diaper 1. Next, the end portions 2L and 3L are pinched in the thickness direction T with a fusion portion-forming apparatus (not shown), and a fusion portion is formed in the end portions 2L and 3L with the fusion portion-forming apparatus. As a result, the end portions 2L and 3L are joined to each other with the fusion portion, and a joining portion 5L is formed. Similarly, the other end 2R of the stomach-side portion 2 in the lateral direction W and the other end 3R of the back-side portion 3 in the lateral direction W are overlapped on each other, and a fusion portion is formed in the end portions 2R and 3R. As a result, the end portions 2R and 3R are joined to each other with the fusion portion, and a joining portion 5R is formed.

In the embodiment according to the present disclosure, the fusion portion-forming apparatus is formed of an ultrasonic generator. That is, ultrasonic energy is applied to the end portions 2L and 3L and the end portions 2R and 3R respectively from the ultrasonic generator, thereby forming the fusion portions.

Next, the joining portions 5L and 5R of the embodiment according to the present disclosure will be further described. The disposable diaper 1 of the embodiment according to the present disclosure is formed to be substantially symmetrically with respect to the lateral direction centerline CW. Therefore, the joining portion 5L and the joining portion 5R have substantially the same configuration. Therefore, hereinafter, the joining portion 5L will be described, and the joining portion 5R will not be described.

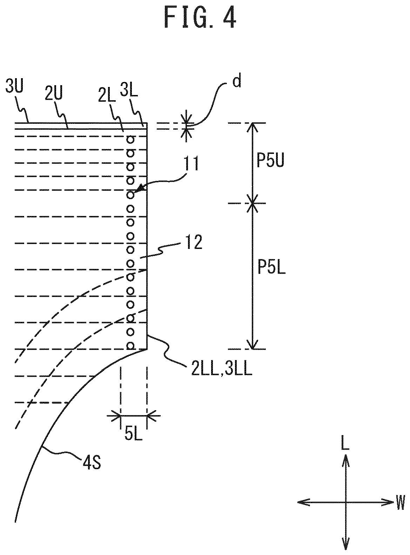

Fig. 4 shows a partial plan view of a disposable diaper 1 according to a reference example in an extended state. As shown in Fig. 4, the end portion 2L of the stomach-side portion 2 in the lateral direction W and the end portion 3L of the back-side portion 3 in the lateral direction W are joined to each other with a plurality of fusion portions 11 aligned at intervals in the longitudinal direction L, thereby forming the joining portion 5L. The stomach-side portion 2 and the back-side portion 3 are offset by d in the longitudinal direction L relative to each other. The offset amount d is, for example, in a range of ±0.5 to 10 mm. In a case where the offset amount d is zero, the stomach-side portion 2 and the back-side portion 3 are not offset relative to each other in the longitudinal direction L. Meanwhile, the outer edge 2LL of the stomach-side portion 2 in the lateral direction W and the outer edge 3LL of the back-side portion 3 in the lateral direction W are aligned relative to each other in the lateral direction W.

The joining portion 5L is divided into an upper joining part P5U positioned on the upper side and a lower joining part P5L positioned on the lower side in the longitudinal direction L. The WG elastic members 6WG, the FT elastic members 6FG, and the LG elastic members 6LG are also provided in the joining portion 5L. Therefore, the joining portion 5L is elastic or contractive in the lateral direction W.

The upper joining part P5U corresponds to the upper regions A2U and A3U. The lower joining part P5L corresponds to the lower regions A2L and A3L. Furthermore, as described above, the tensile stress in the lateral direction W in the upper regions A2U and A3U is larger than the tensile stress in the lateral direction W in the lower regions A2L and A3L. Therefore, tensile stress in the lateral direction W in the upper joining part P5U is larger than tensile stress in the lateral direction W in the lower joining part P5L.

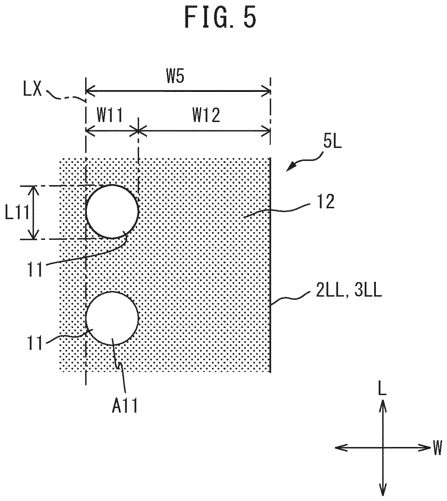

Fig. 5 shows an enlarged view of the joining portion 5L of the embodiment according to the present disclosure. In the embodiment shown in Fig. 5, the joining portion 5L is defined in the lateral direction W between the outer edge 2LL of the stomach-side portion 2 in the lateral direction W and the outer edge 3LL of the back-side portion 3 in the lateral direction W, and a reference line LX extending in the longitudinal direction L. The reference line LX extends in the longitudinal direction L, for example, along the portions of the plurality of fusion portions 11 that are positioned on the innermost side in the lateral direction W. The joining portion 5L is defined in the longitudinal direction L between the upper edge 2U of the stomach-side portion 2 or the upper edge 3U of the back-side portion 3 and the side edge 4S of the intermediate portion 4 (refer to Fig. 4).

As shown in Fig. 5, the joining portion 5L of the embodiment according to the present disclosure includes the above-described fusion portions 11 and a margin 12 present on the outer side of the fusion portions 11 in the lateral direction W. The margin 12 does not include the fusion portions 11. In Fig. 5, W5 represents the dimension of the joining portion 5L in the lateral direction W, W11 represents the dimension of the fusion portion 11 in the lateral direction W, L11 represents the dimension of the fusion portion 11 in the longitudinal direction, W12 represents the dimension of the margin 12 in the lateral direction W, and A11 represents the area of each fusion portion 11, respectively. The dimension of the margin 12 in the lateral direction W is, for example, 10 mm.

In the joining portion 5L of the embodiment according to the present disclosure, as described above, the margin 12 is provided on the outer side of the fusion portions 11 in the lateral direction W. Such a margin 12 is capable of covering the fusion portions 11. This will be described with reference to Fig. 6.

Fig. 6 is a schematic view of the joining portion 5L viewed from above in the longitudinal direction when a wearer wears the disposable diaper 1 for himself or herself or when a caregiver puts the disposable diaper 1 on a dependent. In Fig. 6, WB represents the wearer side or inner side of the disposable diaper 1, and NWB represents the side opposite to the wearer or the outer side of the disposable diaper 1, respectively.

It is considered that the wearer or the caregiver grasps the disposable diaper 1 around the joining portion 5L with, for example, the thumb F1 and the index finger F2 and pulls up the disposable diaper 1. In this case, in the embodiment shown in Fig. 6, the fusion portions 11 are covered with the margin 12. In other words, the margin 12 is interposed between the index finger F2 and the fusion portions 11. As a result, the wearer or the caregiver is restricted from directly touching the relatively hard fusion portions 11. Therefore, the texture of the outside of the disposable diaper 1 is improved. Therefore, the wearer or the caregiver is restricted from feeling discomfort. As described above, the embodiment according to the present disclosure seeks to solve a problem that is caused on the outside of the disposable diaper 1 due to the fusion portions 11 when the wearer wears the disposable diaper 1 by himself, or the caregiver puts the wearer in it. Such a technical idea has not existed thus far. In the embodiment shown in Fig. 6, the back-side portion 3 is interposed between the thumb F1 and the fusion portions 11, and, even on the inside WB of the disposable diaper 1, the wearer or the caregiver is restricted from directly touching the fusion portions 11. Note that the stomach-side portion 2 may be interposed between the thumb F1 and the fusion portions 11.

In addition, in the embodiment according to the present disclosure, the dimension W12 of the margin 12 in the lateral direction in the upper joining part P5U is larger than the dimension W12 of the margin 12 in the lateral direction in the lower joining part P5L. When the dimension W12 of the margin 12 in the lateral direction is large, it is possible to reliably cover the fusion portions 11 with the margin 12. The possibility of the wearer or the caregiver touching the upper joining part P5U is larger than the possibility of the wearer or the caregiver touching the lower joining part P5L. Therefore, in the embodiment according to the present disclosure, the wearer or the caregiver is further restricted from feeling discomfort.

Furthermore, in the embodiment according to the present disclosure, the tensile stress in the lateral direction in the upper joining part P5U is larger than the tensile stress in the lateral direction in the lower joining part P5L. When the tensile stress in the lateral direction is relatively low, the degree of freedom of the margin 12 increases, and there is a concern that the margin 12 may move to a position where the margin 12 does not cover the fusion portions 11. In contrast, in the embodiment according to the present disclosure, since the tensile stress in the lateral direction in the upper joining part P5U is relatively high, the margin 12 is more reliably maintained near the fusion portions 11. In addition, the margin 12 further contracts due to the tensile stress in the lateral direction, whereby the cushioning performance of the margin 12 is enhanced.

Furthermore, in the embodiment according to the present disclosure, the area A11 of each of the fusion portions 11 positioned in the upper joining part P5U is larger than the area A11 of each of the fusion portions 11 positioned in the lower joining part P5L. As a result, the wearer or the caregiver that has touched the joining portions 5L and 5R is restricted from feeling discomfort while maintaining the joining strength in the upper joining part P5U relatively high.

Furthermore, in the embodiment according to the present disclosure, in the upper joining part P5U, the dimension W12 of the margin 12 in the lateral direction is equal to or larger than the dimension W11 of the fusion portion 11 in the lateral direction. As a result, the margin 12 is made larger in the upper joining part P5U. In another embodiment, in the upper joining part P5U, the dimension W12 of the margin 12 in the lateral direction is smaller than the dimension W11 of the fusion portion 11 in the lateral direction. In still another embodiment, in the lower joining part P5L, the dimension W12 of the margin 12 in the lateral direction is equal to or larger than the dimension W11 of the fusion portion 11 in the lateral direction. In still another embodiment, in the lower joining part P5L, the dimension W12 of the margin 12 in the lateral direction is smaller than the dimension W11 of the fusion portion 11 in the lateral direction.

Furthermore, in the embodiment according to the present disclosure, the occupancy of the fusion portions 11 in the upper joining part P5U is smaller than the occupancy of the fusion portions 11 in the lower joining part P5L. The occupancy of the fusion portions 11 in a certain portion is represented by the ratio of the total of the areas A11 of the fusion portions 11 positioned in the certain portion to the area of the certain portion. In such a case, the feeling at the time of touching the upper joining part P5U becomes more flexible. In another embodiment, the occupancy of the fusion portions 11 in the upper joining part P5U is equal to or larger than the occupancy of the fusion portions 11 in the lower joining part P5L.

Furthermore, in the embodiment according to the present disclosure, the dimension W11 of the fusion portion 11 in the lateral direction in the upper joining part P5U is smaller than the dimension W11 of the fusion portion 11 in the lateral direction in the lower joining part P5L. As a result, it becomes possible to make the margin 12 larger in the upper joining part P5U. In another embodiment, the dimension W11 of the fusion portion 11 in the lateral direction in the upper joining part P5U is equal to or larger than the dimension W11 of the fusion portion 11 in the lateral direction in the lower joining part P5L.

Furthermore, in the embodiment according to the present disclosure, the outline of the fusion portion 11 in the upper joining part P5U has a curved shape throughout the entire circumference. As a result, the fusion portion 11 is likely to receive stress not only in the lateral direction W but also in a direction traversing the lateral direction W. Therefore, it becomes possible for the margin 12 to efficiently cover the fusion portions 11. In addition, if there is a straight portion in part or all of the outline of the fusion portion 11, a corner part is included in the outline of the fusion portion 11. As a result, there is a concern that the rigidity of the joining portions 5L and 5R may be increased due to the corner part or there is a concern that a wearer or the like may feel discomfort when touching the joining portions 5L and 5R. When the outline of the fusion portion 11 has a curved shape throughout the entire circumference, such a disadvantage is restricted. In another embodiment, part or all of the outline of the fusion portion 11 in the upper joining part P5U has a straight shape. In still another embodiment, the outline of the fusion portion 11 in the lower joining part P5L has a curved shape throughout the entire circumference. In far still another embodiment, part or all of the outline of the fusion portion 11 in the lower joining part P5L has a straight shape.

Furthermore, in the embodiment according to the present disclosure, in the upper joining part P5U, the dimension L11 of the fusion portion 11 in the longitudinal direction is equal to or smaller than the dimension W11 of the fusion portion 11 in the lateral direction. As a result, the flexibility of the upper joining part P5U in the longitudinal direction is further enhanced. In another embodiment, in the upper joining part P5U, the dimension L11 of the fusion portion 11 in the longitudinal direction is larger than the dimension W11 of the fusion portion 11 in the lateral direction. In still another embodiment, in the lower joining part P5L, the dimension L11 of the fusion portion 11 in the longitudinal direction is equal to or smaller than the dimension W11 of the fusion portion 11 in the lateral direction. In still another embodiment, in the lower joining part P5L, the dimension L11 of the fusion portion 11 in the longitudinal direction is larger than the dimension W11 of the fusion portion 11 in the lateral direction.

Furthermore, in the embodiment according to the present disclosure, the disposable diaper 1 is for adults. Specifically, for example, in a natural state or a contracted state, the ratio of the dimension of the absorbent body 8 in the lateral direction W to the dimension of the disposable diaper 1 in the lateral direction W is 50% or less. The dimension of the disposable diaper 1 in the lateral direction W and the dimension of the absorbent body 8 in the lateral direction W are measured at, for example, an almost midpoint in the longitudinal direction L of the stomach-side portion 2 or the back-side portion 3 in a natural state.

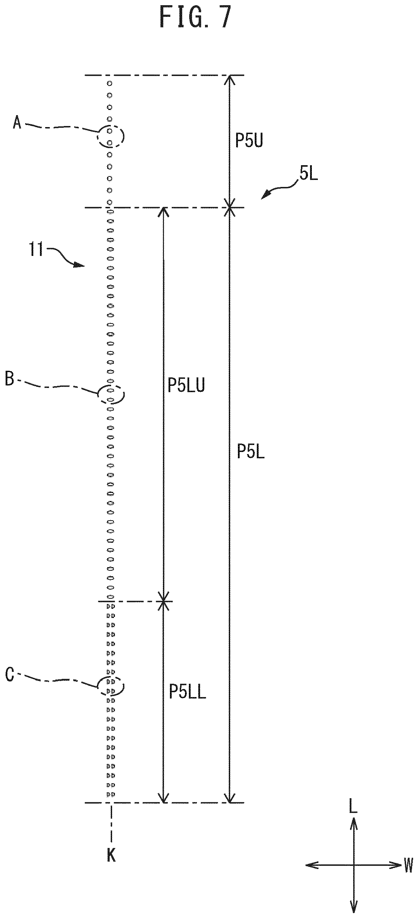

Fig. 7 shows a joining portion 5L of another embodiment according to the present disclosure. In the embodiment shown in Fig. 7, the joining portion 5L is divided into an upper joining part P5U and a lower joining part P5L. In addition, the lower joining part P5L is further divided into an upper small portion P5LU positioned on the upper side and a lower small portion P5LL positioned on the lower side in the longitudinal direction L. In the embodiment shown in Fig. 7, the fusion portions 11 positioned in the upper joining part P5U, the fusion portions 11 positioned in the upper small portion P5LU, and the fusion portions 11 positioned at the lower small portion P5LL are aligned along, for example, a common alignment axis K parallel to the longitudinal direction L.

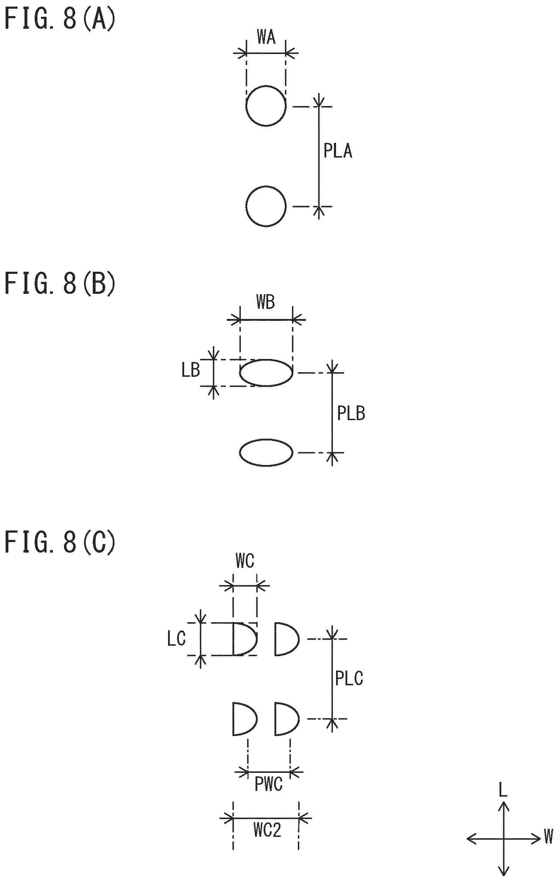

Fig. 8(A) shows the fusion portions 11 positioned in the upper joining part P5U. The fusion portions 11 shown in Fig. 8(A) are aligned in a row in the longitudinal direction L and have a circular shape. The radius of the fusion portion 11 is, for example, 1.43 mm. The pitch PLA between the fusion portions 11 in the longitudinal direction L is, for example, 3.8 mm. In the upper joining part P5U, the fusion portions 11 are aligned in the lateral direction W.

Fig. 8(B) shows the fusion portions 11 positioned in the upper small portion P5LU. The fusion portions 11 shown in Fig. 8(B) are aligned in a row in the longitudinal direction L and have an elliptical shape. The dimension WB of the fusion portion 11 in the lateral direction is, for example, 2.0 mm. The dimension LB of the fusion portion 11 in the longitudinal direction is, for example, 1.0 mm. The pitch PLB between the fusion portions 11 in the longitudinal direction L is, for example, 3.0 mm.

Fig. 8(C) shows the fusion portions 11 positioned in the lower small portion P5LL. The fusion portions 11 shown in Fig. 8(C) are aligned in two rows in the longitudinal direction L and have a long semicircular or semi-elliptical shape. The dimension WC of a single fusion portion 11 in the lateral direction is, for example, 0.8 mm. The dimension LC of a single fusion portion 11 in the longitudinal direction is, for example, 1.2 mm. The pitch PLC between the fusion portions 11 in the longitudinal direction L is, for example, 3.0 mm. The pitch PWC between the fusion portions 11 in the lateral direction W is, for example, 1.5 mm. The dimension WC2 of two fusion portions 11 in the lateral direction is, for example, 2.3 mm. The curved outlines of the fusion portions 11 are disposed, for example, outward in the lateral direction W.

In the embodiment shown in Fig. 7, the variety of numerical values in the lower joining part P5L (for example, the dimension of the margin 12 in the lateral direction) are obtained using the numerical values in the upper small portion P5LU and the numerical values in the lower small portion P5LL. The numerical values in the lower joining part P5L are obtained from, for example, the (simple, weighted) average value, median value, mode value, maximum value, minimum value, and the like of the numerical values in the upper small portion P5LU and the numerical values in the lower small portion P5LL.

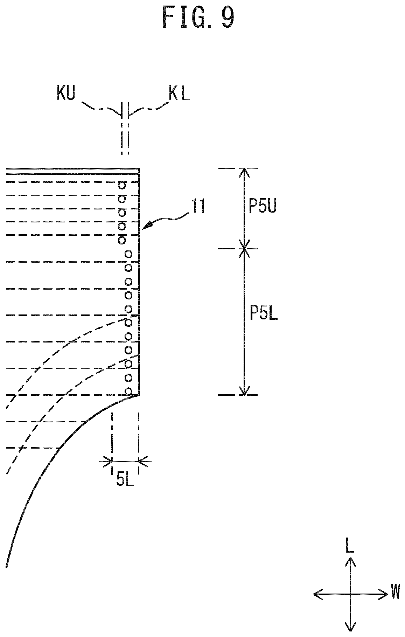

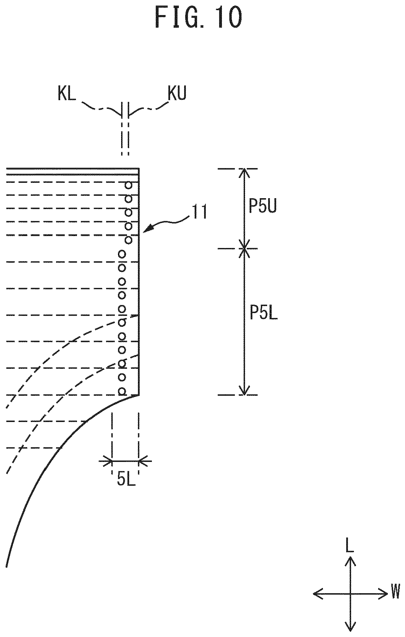

In another embodiment shown in Fig. 9 and in the reference example of Fig. 10, the fusion portions 11 positioned in the upper joining part P5U are aligned along an alignment axis KU extending in the longitudinal direction L, and the fusion portions 11 positioned in the lower joining part P5L are aligned along an alignment axis KL extending in the longitudinal direction L. In addition, in the embodiment shown in Fig. 9, the alignment axis KU is positioned on the inner side of the alignment axis KL in the lateral direction W. In the reference example shown in Fig. 10, the alignment axis KU is positioned on the outer side of the alignment axis KL in the lateral direction W.

In still another embodiment according to the present disclosure, the outline of the fusion portion 11 positioned in a region occupying the upper 2/3 part of the joining portion 5L or 5R in the longitudinal direction L has a curved shape throughout the entire circumference. When there is a straight portion in part or all of the outline of the fusion portion 11, a corner part is included in the outline of the fusion portion 11. As a result, there is a concern that the rigidity of the joining portion 5L or 5R may be increased due to the corner part or there is a concern that a wearer or the like may feel discomfort when touching the joining portion 5L or 5R. When the outline of the fusion portion 11 has a curved shape throughout the entire circumference, such a disadvantage is restricted.

In the variety of embodiments according to the present disclosure described above, both ends of the stomach-side portion 2 and the back-side portion 3 in the lateral direction are joined to each other with a pair of the joining portions 5L and 5R. In another embodiment (not shown), ends on one side of the stomach-side portion 2 and the back-side portion 3 in the lateral direction W are joined to each other with a joining portion, and ends on the other side of the stomach-side portion 2 and the back-side portion 3 in the lateral direction W are joined to each other by an approach different from the joining portion.

### [Reference Signs List]

1 Disposable diaper
2 Stomach-side portion
3 Back-side portion
5L, 5R Joining portion
11 Fusion portion
12 Margin
P5U Upper joining part
P5L Lower joining part

## Claims

1. An absorbent article (1) comprising:
a stomach-side portion (2) and a back-side portion (3) that are joined to each other with at least one joining portion (5L, 5R),
wherein the joining portion (5L, 5R) is formed by joining, with a plurality of fusion portions (11), an end portion (2L) of the stomach-side portion (2) in a lateral direction (W) and an end portion (3L) of the back-side portion (3) in the lateral direction (W) that are overlapped on each other such that inner surfaces face each other, the plurality of fusion portions (11) being aligned in a longitudinal direction (L) at intervals,
each of the joining portion (5L, 5R) is divided into an upper joining part (P5U) positioned on an upper side and a lower joining part (P5L) positioned on a lower side in the longitudinal direction (L),
the joining portion (5L, 5R) is elastic in the lateral direction (W), tensile stress in the lateral direction (W) in the upper joining part (P5U) is larger than tensile stress in the lateral direction (W) in the lower joining part (P5L),
the joining portion (5L, 5R) includes the fusion portions (11) and a margin (12) present on an outer side of the fusion portions (11) in the lateral direction (W),
a dimension of the margin (12) in the lateral direction (W) in the upper joining part (P5U) is larger than a dimension of the margin (12) in the lateral direction (W) in the lower joining part (P5L), and
an area of each of the fusion portions (11) positioned in the upper joining part (P5U) is larger than an area of each of the fusion portions (11) positioned in the lower joining part (P5L).

2. The absorbent article (1) according to claim 1, wherein, in the upper joining part (P5U), the dimension of the margin (12) in the lateral direction (W) is equal to or larger than a dimension of the fusion portion (11) in the lateral direction (W).

3. The absorbent article (1) according to claim 1 or 2, wherein an occupancy of the fusion portions (11) in the upper joining part (P5U) is smaller than an occupancy of the fusion portions (11) in the lower joining part (P5L), wherein the occupancy of the fusion portions (11) in the upper joining part (P5U) is the ratio of the total areas of the fusion portions (11) positioned in the upper joining part (P5U) to the area of the upper joining part (P5U), and the occupancy of the fusion portions (11) in the lower joining part (P5L) is the ratio of the total areas of the fusion portions (11) positioned in the lower joining part (P5L) to the area of the lower joining part (P5L).

4. The absorbent article (1) according to any one of claims 1 to 3, wherein a dimension of the fusion portion (11) in the lateral direction (W) in the upper joining part (P5U) is smaller than a dimension of the fusion portion (11) in the lateral direction (W) in the lower joining part (P5L).

5. The absorbent article (1) according to any one of claims 1 to 4, wherein an outline of the fusion portion (11) in the upper joining part (P5U) has a curved shape throughout an entire circumference.

6. The absorbent article (1) according to any one of claims 1 to 5, wherein, in the upper joining part (P5U), a dimension of the fusion portion (11) in the longitudinal direction (L) is equal to or smaller than a dimension of the fusion portion (11) in the lateral direction (W).

7. The absorbent article (1) according to any one of claims 1 to 6, wherein the absorbent article (1) is for adults.

## Patentansprüche

1. Absorbierender Gegenstand (1), umfassend:
einen magenseitigen Abschnitt (2) und einen rückenseitigen Abschnitt (3), die mit wenigstens einem Verbindungsabschnitt (5L, 5R) miteinander verbunden sind,
wobei der Verbindungsabschnitt (5L, 5R) durch Verbinden mit einer Mehrzahl von Fusionsabschnitten (11) eines Endabschnitts (2L) des magenseitigen Abschnitts (2) in einer lateralen Richtung (W) und eines Endabschnitts (3L) des rückenseitigen Abschnitts (3) in der lateralen Richtung (W), die aufeinander überlappt werden, sodass innere Oberfläche einander zugewandt sind, wobei die Mehrzahl von Fusionsabschnitten (11) in einer Längsrichtung (L) in Intervallen ausgerichtet sind,
wobei jeder der Verbindungsabschnitts (5L, 5R) in einen oberen Verbindungsteil (P5U), der auf einer oberen Seite positioniert ist, und einen unteren Verbindungsteil (P5L), der auf einer unteren Seite positioniert ist, in der Längsrichtung (L) geteilt wird,
der Verbindungsabschnitt (5L, 5R) in der lateralen Richtung (W) elastisch ist, die Dehnspannung in der lateralen Richtung (W) in dem oberen Verbindungsteil (P5U) größer ist als die Dehnspannung in der lateralen Richtung (W) in dem unteren Verbindungsteil (P5L),
der Verbindungsabschnitt (5L, 5R) die Fusionsabschnitte (11) und einen Saum (12) beinhaltet, der auf einer äußeren Seite der Fusionsabschnitte (11) in der lateralen Richtung (W) vorhanden ist,
eine Abmessung des Saums (12) in der lateralen Richtung (W) in dem oberen Verbindungsteil (P5U) größer ist als eine Abmessung des Saums (12) in der lateralen Richtung (W) in dem unteren Verbindungsteil (P5L), und
eine Fläche jedes der Fusionsabschnitte (11), die in dem oberen Verbindungsteil (P5U) positioniert sind, größer ist als eine Fläche jedes der Fusionsabschnitte (11), die in dem unteren Verbindungsteil (P5L) positioniert sind.

2. Absorbierender Gegenstand (1) nach Anspruch 1, wobei in dem oberen Verbindungsteil (P5U) die Abmessung des Saums (12) in der lateralen Richtung (W) gleich oder größer als eine Abmessung des Fusionsabschnitts (11) in der lateralen Richtung (W) ist.

3. Absorbierender Gegenstand nach Anspruch 1 oder 2, wobei eine Belegung der Fusionsabschnitte (11) in dem oberen Verbindungsteil (P5U) kleiner ist als eine Belegung der Fusionsabschnitte (11) in dem unteren Verbindungsteil (P5L), wobei die Belegung der Fusionsabschnitte (11) in dem oberen Verbindungsteil (P5U) das Verhältnis der gesamten Flächen der Fusionsabschnitte (11), die in dem oberen Verbindungsteil (P5U) positioniert sind, zu der Fläche des oberen Verbindungsteils (P5U) ist, und die Belegung der Fusionsabschnitte (11) in dem unteren Verbindungsteil (P5L) das Verhältnis der gesamten Flächen der Fusionsabschnitte (11), die in dem unteren Verbindungsteils (P5L) positioniert sind, zu der Fläche des unteren Verbindungsteils (P5L) ist.

4. Absorbierender Gegenstand (1) nach einem der Ansprüche 1 bis 3, wobei eine Abmessung des Fusionsabschnitts (11) in der lateralen Richtung (W) in dem oberen Verbindungsteil (P5U) kleiner ist als eine Abmessung des Verbindungsabschnitts (11) in der lateralen Richtung (W) in dem unteren Verbindungsteil (P5L).

5. Absorbierender Gegenstand (1) nach einem der Ansprüche 1 bis 4, wobei ein Umriss des Fusionsabschnitts (11) in dem oberen Verbindungsteil (P5U) eine gekrümmte Form in einem gesamten Umfang aufweist.

6. Absorbierender Gegenstand (1) nach einem der Ansprüche 1 bis 5, wobei in dem oberen Verbindungsteil (P5U) eine Abmessung des Fusionsabschnitts (11) in der Längsrichtung (L) gleich oder kleiner als eine Abmessung des Fusionsabschnitts (11) in der lateralen Richtung (W) ist.

7. Absorbierender Gegenstand (1) nach einem der Ansprüche 1 bis 6, wobei der absorbierende Gegenstand für Erwachsene ist.

## Revendications

1. Article absorbant (1) comportant :
une partie côté estomac (2) et une partie côté dos (3) qui sont assemblées l'une par rapport à l'autre au moyen d'au moins une partie d'assemblage (5L, 5R),
dans lequel la partie d'assemblage (5L, 5R) est formée en assemblant, au moyen d'une pluralité de parties de fusion (11), une partie d'extrémité (2L) de la partie côté estomac (2) dans une direction allant dans le sens latéral (W) et une partie d'extrémité (3L) de la partie côté dos (3) dans la direction allant dans le sens latéral (W) qui se chevauchent l'une l'autre de telle sorte que les surfaces intérieures sont orientées l'une vers l'autre, les parties de fusion de la pluralité de parties de fusion (11) étant alignées dans une direction allant dans le sens longitudinal (L) selon des intervalles,
chacune des parties d'assemblage (5L, 5R) est divisée en une partie d'assemblage supérieure (P5U) se trouvant sur un côté supérieur et une partie d'assemblage inférieure (P5L) se trouvant sur un côté inférieur dans la direction allant dans le sens longitudinal (L),
la partie d'assemblage (5L, 5R) est élastique dans la direction allant dans le sens latéral (W), la contrainte de traction dans la direction allant dans le sens latéral (W) dans la partie d'assemblage supérieure (P5U) est supérieure à la contrainte de traction dans la direction allant dans le sens latéral (W) dans la partie d'assemblage inférieure (P5L),
la partie d'assemblage (5L, 5R) comprend les parties de fusion (11) et une marge (12) présente sur un côté extérieur des parties de fusion (11) dans la direction allant dans le sens latéral (W),
une dimension de la marge (12) dans la direction allant dans le sens latéral (W) dans la partie d'assemblage supérieure (P5U) est supérieure à une dimension de la marge (12) dans la direction allant dans le sens latéral (W) dans la partie d'assemblage inférieure (P5L), et
une surface de chacune des parties de fusion (11) se trouvant dans la partie d'assemblage supérieure (P5U) est supérieure à une surface de chacune des parties de fusion (11) se trouvant dans la partie d'assemblage inférieure (P5L).

2. Article absorbant (1) selon la revendication 1, dans lequel, dans la partie d'assemblage supérieure (P5U), la dimension de la marge (12) dans la direction allant dans le sens latéral (W) est égale ou supérieure à une dimension de la partie de fusion (11) dans la direction allant dans le sens latéral (W) .

3. Article absorbant (1) selon la revendication 1 ou la revendication 2, dans lequel une occupation des parties de fusion (11) dans la partie d'assemblage supérieure (P5U) est inférieure à une occupation des parties de fusion (11) dans la partie d'assemblage inférieure (P5L), dans lequel l'occupation des parties de fusion (11) dans la partie d'assemblage supérieure (P5U) est le rapport des surfaces totales des parties de fusion (11) se trouvant dans la partie d'assemblage supérieure (P5U) par rapport à la surface de la partie d'assemblage supérieure (P5U), et l'occupation des parties de fusion (11) dans la partie d'assemblage inférieure (P5L) est le rapport des surfaces totales des parties de fusion (11) se trouvant dans la partie d'assemblage inférieure (P5L) par rapport à la surface de la partie d'assemblage inférieure (P5L).

4. Article absorbant (1) selon l'une quelconque des revendications 1 à 3, dans lequel une dimension de la partie de fusion (11) dans la direction allant dans le sens latéral (W) dans la partie d'assemblage supérieure (P5U) est inférieure à une dimension de la partie de fusion (11) dans la direction allant dans le sens latéral (W) dans la partie d'assemblage inférieure (P5L).

5. Article absorbant (1) selon l'une quelconque des revendications 1 à 4, dans lequel un contour de la partie de fusion (11) dans la partie d'assemblage supérieure (P5U) a une forme courbe au niveau de toute une circonférence.

6. Article absorbant (1) selon l'une quelconque des revendications 1 à 5, dans lequel, dans la partie d'assemblage supérieure (P5U), une dimension de la partie de fusion (11) dans la direction allant dans le sens longitudinal (L) est égale ou inférieure à une dimension de la partie de fusion (11) dans la direction allant dans le sens latéral (W).

7. Article absorbant (1) selon l'une quelconque des revendications 1 à 6, dans lequel l'article absorbant (1) est destiné à des adultes.
